# EUROPEAN PATENT APPLICATION

(11) **EP 1 536 019 A1**
(43) Date of publication of application: **01.06.2005**
(21) Application number: 03787581.2
(22) Date of filing: 19.08.2003
(51) Int. Cl.: C12Q 1/25, C12Q 1/68

(54) **USING A POLYMERASE WITH 3' TO 5' EXONUCLEASE ACTIVITY TO PERFORM GENE SEQUENCE ANALYSIS**

(30) Priority: 19.08.2002 CN 02129166
(71) Applicant: Zhang, Xu, Tianjin 300192 (CN)
(72) Inventor: LI, Kai, Nankai District, Tianjin 300191 (CN)
(74) Representative: Dey, Michael, Dr.
(86) International application number: PCT/CN2003/000695
(87) International publication number: WO 2004/016806

(57) **Abstract**

A method for gene sequence determination by utilizing polymerases with 3'to 5'exonuclease activity is disclosed, which comprises the following steps: (1) preparing sets of specific oligonucleotide primers with their 3 'termini being modified, (2) performing a selective primer extension with a molecular "on/off" switch consisting of polymerases with 3'to 5'exonuclease activities and the modified primers, and (3) visualizing the products of the primer extension reaction to determine the gene sequence. The fidelity of the molecular "on/off" switch employed in the present invention is higher than in the conventional primer extension reaction wherein polymerases without proofreading activity are employed. The present method can also be used to sequence an unknown sequence directly, and does not depend on a known segment of sequence for the sequencing. In this method, each signal obtained contains the sequence information of one or more nucleotides. Using this method, single nucleotide polymorphism assay and gene sequence analysis can be performed in high throughput screening.

## Description

### Technical Field

this method is for sequence determination, in specific, this method provides a rapid sequence determination by using the combination of destroying ability of polymerases with 3'to 5'exnuclease and the nuclease-resistant feature of allele specific primers inherited from chemical modifications.

### Background:

At the moment, sequencing analysis uses polymerases without 3' to 5'exonuclease activity for primer extension. And each signal detected only provides the information of one nucleotide. The most significant problem is the routine sequencing is not compatible for high thoughtput analysis of single nucleotide polymorphism. Although the current sequencing method has been successfully used in genome sequencing analysis, cost-efficient, and rapid sequencing method is the research hot topic in biotechnological development, such as Affymetrix's oligo microarray that does have been used in research.

The typical sequence analysis is Sanger's enzyme method based on terminal termination with ddNTP, which is the main method in modern sequencing analysis. This method is very reliable. But each signal from this method only provides information of one nucleotide, which restrains its efficiency. One substantial problem of this classic sequencing method is that it requires a short sequences that has been known for primer design. The dependence on known sequences greatly slowed down the speed for unknown sequence determination. In addition, the classical sequencing method is not high in efficiency as it needs a premier amplification of the samples to be sequenced. Obviously, this method is time-consuming, and is a indirect sequencing analysis and cannot be used for de novo sequencing.

Oligonucleotide microarray can be used for sequencing too. However, the heterogeneity of hybridization determines its accuracy, which is currently used for laboratory research. The practical application of this method is to be evaluated with time.

Additionally, all sequencing methods including classical terminal termination and recently developed single base extension all employ terminator, labeled dideoxinucleotides (ddNTP). Because of ddNTP cannot be further dehydrated for forming a bond with the continuing primer extension, thus its integration causes the termination of primer extension. Therefore, this kind of chemical reaction has a linear relationship with the cycles of the reaction, or products = 2n here n is the cycles used. On the contrary, polymerase chain reaction (PCR) without the terminator (ddNTP) is more efficient as it is exponential related to the reaction cycles (product =n²).

### Disclosure of the Invention

The goal of this invention is to overcome the drawbacks of conventional techniques in order to provide a novel sequence determination method using polymerases having 3'to 5'exonuclease activity. With this invention, both single nucleotide polymorphism and unknown sequences can be determined (SNP assay and de novo sequencing).

The technical procedures of this invention included:

A method utilizing the high fidelity polymerases with 3'to 5'exonuclease activity for sequence determination, including the following steps:
1). allele specific primers with their 3'termini specifically modified,
2). performing a selective primer extension for the discrimination of matched and mismatched amplicons by using a novel "on/off" switch consisting the polymerases with 3'to 5'exonucleases activities to destroy primers from 3'to 5'and primers with exonuclease-resistant characters inherited from specific chemical modification,
3). sequence determination by visualization of the primer extended products.

The primers used in this method contain a set of primers differing in their 3'termini.

The set of primers contains a subset of primers with chemical modification and a subset of primers without chemical modification. When primers contain labels from chemical modification, their products are labeled too when the amplicons are matched. For the unlabeled primers, visualization can use routine methods or using additional labeling by adding labeled dNTP.

The set of primers contains a subset of primers with chemical modification by which the primers are 3'to 5'exonuclease resistant, thus the polymerases with 3'to 5'exonuclease activity cannot destroy the mismatched primers and failed to proofread the mismatched primers, therefore failed to continue primer extension.

Thus, the molecular "on/off" switch can keep or remove the labels in the primers that are sensitive to exonuclease, and can turn off or turn on the primer extension for the exonuclease-resistant primers.

This invention has the following advantages:
1). This method uses the molecular "on/of" switch consisting of polymeases with 3'to 5'exonuclease activity and chemical modified primer set to perform primer extension. Its fidelity is higher than regular primer extension based on the high fidelity proofreading polymerases;
2). This method can be used for de novo sequencing;
3). This sequencing determination can be done without knowing the sequencing;
4). In this method, each signal obtained can decode more than one nucleotide;
5). Using this method, SNP assay can be performed in high throughput screening.

### Explanation of the Drawings

Figure 1 is the illustration of basic principle of this invention;
Figure 2 is the strategy used for sequencing assay using the "on/off" switch;
Figure 3 is the gel electrophoresis picture from example 1;
Figure 4 is the sequencing fig used in example 1;
Figure 5 is the figure showing the liquid scintillation counting number used in example 2;
Figure 6 is the electrophoresis picture in example 3;
Figure 7 is the electrophoresis picture in example 4;
Figure 8 is the electrophoresis picture in example 5;
Figure 9 is the electrophoresis picture in example 5;
Figure 10 is the illustration showing how the molecular "on/off" switch works.

### Detailed Description of the Invention

Combined with the figures, the detailed explanation of the application examples is given below:

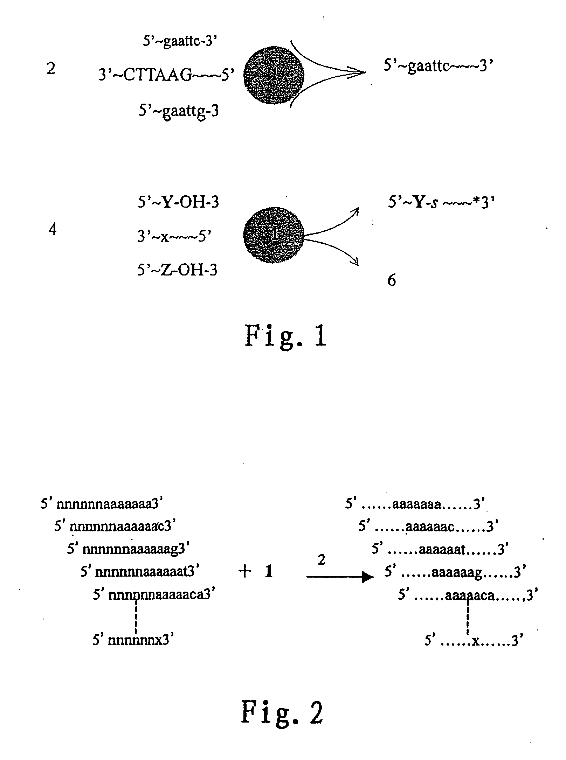

In figure 1, 1 represents the primer extension mediated by polymerases with 3'to 5'exonuclease activity; 2 represents regular primers; 4 represents specifically chemically modified primers; 6 represents the failure of obtaining products or failure to obtain labeled products.

In figure 2, 1 represents target with unknown sequences; 2 represents the primer extension mediated by polymerases with 3'to 5'exonuclease acitivity.

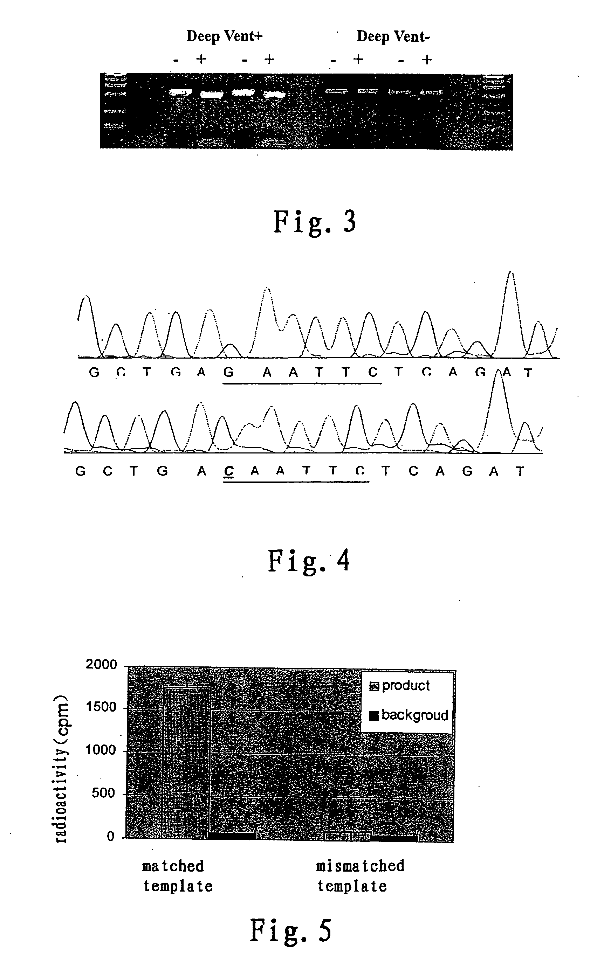

In figure 3, - represents products without EcoR I digestion; + represents products after EcoR I digestion;

In figure 5, product represents products; background represents background signals.

In figure 6, 1,2,3,4,5,6,7,8,9,10, 11, 12 represents different annealing temperature, they were 46, 48, 50, 52, 54, 56, 58, 60, 62, 64, and 66 °C respectively.

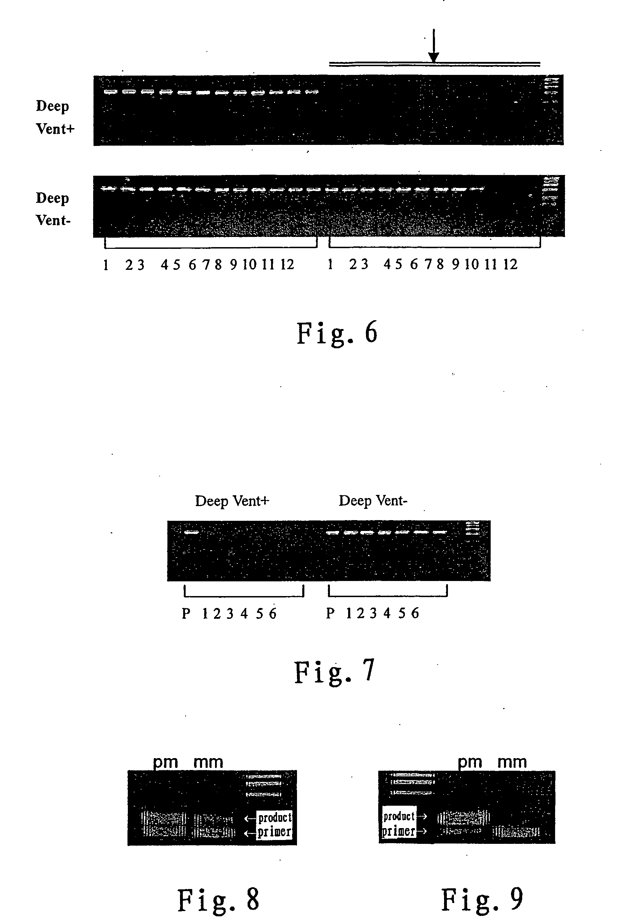

In figure 7, P represents matched primers; 1,2,3,4,5,6 represents mismatched primers at -1, -2, -3, -4, -5 and -6 upstream the 3'terminus.

In figures 8 and 9, PM represents matched primer; MM represents mismatched primer;

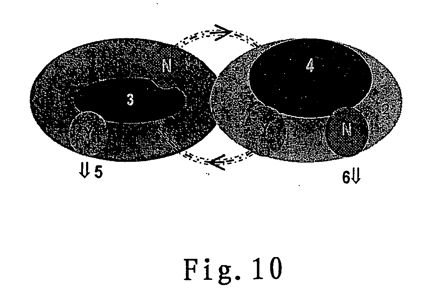

In figure 10, 1 represents polymerase domain, 2 represents the 3'to 5'exonuclease domain, 3 represents product, 4 represents mismatch proofreading process, 5 represents the "on" status of the "on/off' switch, 6 represents the "off" status of the on/off switch; Y represents the replication complex passed the match testing by the polymerase, N represents the failure of the matching testing that triggers the proofreading process then.

This invention employs the novel "on/off" molecular switch consisting of polymerases with 3' to 5' exonuclease function and allele specific primers with their 3' terminal modified for primer extension in order to determine sequence information of template to be analyzed according to the match status: perfectly matched or not completely matched between the allele specific primer set and the template to be assayed. During the procedure of primer extension, the method from this invention directly decodes the sequence information at and upstream the 3' termini from the proofreading activity of the high fidelity polymerases, thus one or more than one nucleotides are sequenced , which offers method for single nucleotide assay for known sequences and de novo sequencing for unknown sequences.

As to the first newly integrated nucleotides after its initiation step, polymerases without proofreading activity has a mutation rate of 2/1000. However, this mutation rate can be decreased by 80-90 % when using polymerases with 3' to 5' exonuclease activities in primer extension. This significant difference is well known in DNA replication in plant, animal, and bacteria.

One missing point that is even more significant, occurs at the initiation step between primer extension using polymerases with and without proofreading activities. At the initiation step, whether primer sequences are altered or not is determined. For primer extension using polymerases without proofreading activity, the sequences of primers are 100% not changed no matter the primers are matched with the template or not. Therefore, the products from this kind of primer extension not necessarily truthfully reflect the sequence information of the template. On the contrary, the primer extension using polymerases with 3' to 5' exonuclease activity is different according to the match status of the primer with the template: the primer sequences are not changed if they are matched with template; or the mismatched primers are subjected to exonuclease digestion and changed to matched primer before being extended. Therefore, if the polymerases with 3' to 5' exonuclease activity are well designed and employed, it can be used for sequencing determination.

Regarding to the chemical reactions combined with two different reaction systems, the key point is that those different reactions need to be compatible in their reaction conditions. We studied the primer extension using polymerases with 3' to 5' exonuclease activities and found that high fidelity polymerases can easily discriminate the matched and mismatched amplicons in a broad range of annealing temperatures from 46 to 66 °C. Since the proofreading activity is negatively associated with the concentrations of dNTP, the discrimination of perfectly matched and mismatched amplicons are based on the more or less products from primer extension when 1- to 2-fold dNTP is used. When dNTP concentrations lowered to 0.2 to 0.8 fold of normal concentration, the difference is more significant and the discrimination of the matched and mismatched amplicons can be told by the presence or absence of the primer extended products.

Another key point of reasonably application of polymerases with 3' to 5' exonuclease activity is how to transform the accurate recognition of template sequences into detectable signals for high throughput screening assay. This invention approaches this goal by (1) label exonuclease-sensitive primers' 3' termini in order to yield two types of primer extended products after proofreading genotyping: products from the matched amplicons inherited labels from primers and labels were digested by proofreading procedure for products from mismatched amplicons; and (2) preparing exonuclease-resistant primers through chemical modification, by which the matched amplicon extended as no exonuclease digestion is processed; but mismatched primers were sent from polymerization domain to exonuclease domain for exonuclease digestion. The exonuclease-resistant property of the primers greatly delayed the proofreading procedure or blocked the procedure, leading to the off switch effect and no primer extended products obtained then. (see figure 1).

With extensive experiments, we observed that matched amplicons are extended regardless the types of polymerases used or the sensitive or resistant to the exonuclease of the primers. However, for the mismatched amplicons, the type and the amount of extended products depend on the fidelity of polymerases used as well as the resistance of primers to exonuclease.

Therefore, a premature termination occurs when exonuclease-resistant primers are extended by polymerases with 3' exonuclease activity. This premature termination is intensified by the phosphorothioate or other modifications that offer exonuclease resistance to primers, resulting in a complete termination of primer extension reaction, which thus forms an "off" switch useful for SNP assay. The allele specific primers is subjected to an initial "on/off" switch screening by the two enzymatic domains of polymerase domain and exonuclease domain that is apart from each other about 3 nm: the matched primers are directly polymerized within the polymerization domain, or by the "on" switch as illustrated in figure 10; whereas mismatched primers are transferred from the polymerization domain to the exonuclease domain where a long time or never-finished digestion procedure is processed due to the exonuclease-resistant property of the primers inherited from chemical modification, leading to the empty polymerization domain during the primer extension reaction, which thus turns "off" DNA polymerization as illustrated in figure 10. This kind of final results of matched primer extended and of mismatched primers did not extend is a yes or no phenomenon, well fulfilled the requirement of single nucleotide polymorphisms.

In addition, the exonuclease sensitive primers with detectable labels yield two types of products: one type with detectable signals and another without, which practically functions as an "on/off" switch at the signal detection point of view.

This novel molecular on/off switch suitable for sequencing analysis has significant theoretical and practical impact in studying SNP.

The detectable signals in the method of this invention vary according to the label methods used. Three methods are different as the following: label the 3 terminal of allele specific primers, label the dNTP, and label the pairing primers.

When labeled dNTP is used, the labeled and unlabeled dNTP varies between 1:10 to 1:100.

When pairing primers are used as the label resources, labeling can be done their 5' termini, or any nucleotide other than 3' termini.

When primers are exonuclease-resistant, visualization is to detect the presence or absence of primer extended products. The presence of the products are visualized as usual methods such as using ethidium bromide or Syber green or other staining methods.

### 1). Preparation of primer set with 3' terminal modifications

Primers are targeting the DNA sequences to be analyzed. Its design includes several situations:
(1). Primers for SNP assay: in general, each allele needs two allele specific forward primers and one reverse primer for pairing. The total number of primer set depending on the practical needs. The two allele specific forward primers are perfectly matched or mismatched with the template respectively, forming a primer subset. Label methods depend on the visualization condition. Primers are synthesized by DNA synthesizer from commercial vendors.
   For example, the following primers are designed: 
Forward primer: 5' XXXXXXXXXXXXX3'
5' XXXXXXXXXXXXY3'
reverse primer: 5' XXXXXXXXXXXXX3'
 X represents match with template, Y represents mismatch with template, X,Y represent nucleotide after modification.
(2). Primer design for sequencing unknown sequences: a mathematical model using the polymerases with 3' to 5' exonuclease activity for de novo sequence determination. The size of the primers depends on the number of gene specific nucleotides, consisting 4ⁿ primers with 5' degenerated sequences and 3' sequence specific primers. For example, the following primer set has two sequence specific nucleotides at the 3', thus containing 4² primers or 16 primers (X represents degenerate nucleotides).

Xxxxxxxxaa
Xxxxxxxxag
Xxxxxxxxac
Xxxxxxxxat
Xxxxxxxxta
Xxxxxxxxtg
Xxxxxxxxtc
Xxxxxxxxtt
Xxxxxxxxca
Xxxxxxxxcg
Xxxxxxxxcc
Xxxxxxxxct
Xxxxxxxxga
Xxxxxxxxgg
Xxxxxxxxgc
Xxxxxxxxgt

Sequence analysis is based on the alignment or bridging between neighboring primers having several nucleotides identical (see figure 2).
2). To perform primer extension using molecular on/off switch consisting of polymerases with 3' to 5' exonuclease activity and chemically modified primers. In this method, reaction condition is not significantly different from regular primer extension.
3). Visualization of primer extended products and sequence analysis.

Before visualization and detection, the labels attached to DNA molecule shall be separated from the labels which do not attach to DNA molecule. The separation method depends on the specific requirements. When electrophoresis is used in the present invention, the separation stage may be omitted. When biochip is used in the present invention, the washing under stringent condition will clear off the labels not attached to DNA molecule. If the label signal comes from the labeled primers and the background noise is too high, then it may be incubated with low concentrations of exonuclease for 15 min to 1hr at 25-37°C. The label signal of DNA molecule can be visualized and detected by conventional methods. Said conventional method is specifically dependent on the labeling method. If an enzyme label or other chemical label such as digoxin and biotin is used, a specific chemical visualization shall be performed before signal detection.

At visualization and detection stage, if the labeling method (e.g.sulfurization) does not permit the direct scanning detection, then ethidium bromide or GYBR staining method may be used, followed by result analysis on electrophoresis gel, or mass spectroscopy may be used to analyze the results. If the labels are radioactive or direct fluorscene, the visualization step may be omitted as the products can be directly scanned to detect the radioactivity intensity or fluorscene intensity, or can be autographed followed by image scanning analysis. In case of chemoluminescene or indirect fluorescence, the pre-blocking, visualization reaction and washing shall be done before signal detection.

The signal detected reflects the nucleotide sequence information of 3' termini. Sequence analysis can be performed manually or by computerized programming. To determine a single allele is homozygo or heterozygo, the following primers can determine the genotypes. For example:

Forward primer: 5' XXXXXXXXXXXXXX3'
5' XXXXXXXXXXXXXY3'
Reverse primer: 5' XXXXXXXXXXXXXX3'

X represents matched primer (wild type), Y represents mismatched primer (polymorphism genotype or mutated genotype), X, Y represents unmodified and X,Y represents modified nucleotides respectively.

If 5' XXXXXXXXXXXX3' is used for primer extension with wild type template, products are obtained or products with labels are obtained; and no products or no labeled products are obtained using 5' XXXXXXXXXXXY3' for primer extension, indicating that this allele is a X homozygo genotype If 5' XXXXXXXXXXXY3' is used for primer extension with wild type template, products are obtained or products with labels are obtained; and no products or no labeled products are obtained using 5' XXXXXXXXXXXX3' for primer extension, indicating that this allele is a Y homozygo genotype. If both primers yield products, the genotype of the template is heterozygo.

This invention provides a new sequencing method. Its advantage is direct, sensitive, simple, and rapid. This method does not need to previous amplification for sequence analysis, and it therefore is a direct sequencing method. In addition, the high reliability and sensitivity of this method allows rapid sequence analysis to be possible.

### Example 1.

Application of Deep Vent + polymerase that has 3' to 5' exonuclease activity and Deep Vent- polymerase that does not have 3' to 5' exonuclease activity in primer extension, matched and mismatched primers are tested. This experiment is used to illustrate the importance of proofreading activity in sequence analysis (see figures 3 and 4).
1). Primer design:
   Using a 217 bp fragment from mouse genome with the following sequences: cccaagatat ctgagaattc tcagcagcct tccatttaga agggtgttgt tgtctctgag gcaaaaccac atttcttacc gcacaactag agactgagac cagtttctct cattgtcatt gctgctcaga gccagcagaa aagcactcat gacacacact tagaataata gtgcatctga gccaggactg cccttggggt ccattcagct gtttc. Design a forward primer set and a reverse primer set targeting this sequence. The size of the product from these primers is 217 bp. The product also harbors a EcoR I site.
   The matched forward primer is: AGT CCT CTC CTA TCC CAA GAT ATC TGA GAA TTC Mismatched forward primer is: AGT CCT CTC CTA TCC CAA GAT ATC TGA CAA TTC Reverse primer is: 5'gaaacagctgaatggacccaa3'
   These primers were synthesized by MWG company in USA.
2). Primer extension reaction: primer extension was performed using Deep Vent+ and Deep Vent- respectively with the allele specific forward primers and reverse primer. The reaction conditions were: denature at 95 °C for 5 minutes, then cycled 30 times with denature at 95 °C for 10 seconds, annealing at 56 °C for 30 seconds, extension 72 °C for 1 second.
3). Results: PCR results were under electrophoresis with 2.5% agarose gel containing ethidium bromide under 10V/cm direct current, with 0.5 X TBE butter. As shown in Figure 3, matched primers yielded correct-sized products digestible by EcoR I enzyme. However, mismatched primers yielded two types of products depending on the polymerases used: products by polymerases with 3' to 5' exonuclease activity were EcoR I digestible but products by Deep Vent- were EcoR I resistant. These products were further sequenced, as shown in figure 4: products from Deep Vent + had the sequences of 5'AGT CCT CTC CTA TCC CAA GAT ATC TGA *G*AA TTC........TTGGGTCCATTCAGCTGTTTC 3'; whereas, products from Deep Vent-had the sequences of 5'AGT CCT CTC CTA TCC CAA GAT ATC TGA *C*AA TTC........TTGGGTCCATTCAGCTGTTTC 3'.

These results demonstrated that polymerases without 3' to 5' proofreading activity did not change the sequences of the primers (error or false positives in genotyping test); however, polymerases with 3' to 5' exonuclease activity can proofread the mismatched nucleotides at primers in order to reconstruct a matched amplicon before their extension, thus the primer's sequences were altered. This proofreading function can be used for decoding the sequences of template to be assayed when properly used with specific algorithm.

### Example 2.

SNP detection using polymerases with 3' to 5' exonuclease activity together with the isotope labeled matched and mismatched primers.
1). Primer design:
   Using a 217 bp fragment from mouse genome with the following sequences: cccaagatat ctgagaattc tcagcagcct tccatttaga agggtgttgt tgtctctgag gcaaaaccac atttcttacc gcacaactag agactgagac cagtttctct cattgtcatt gctgctcaga gccagcagaa aagcactcat gacacacact tagaataata gtgcatctga gccaggactg cccttggggt ccattcagct gtttc. Design a forward primer set and a reverse primer set targeting this sequence. The size of the product from these primers is 217 bp. There were two forward primers, a unlabeled primer with the sequence of 5'atcccaagatatctgagaatt3' and a 3' terminal 3[H] labeled primer with the identical sequence of 5'atcccaagatatctgagaatT3'; reverse primers were not labeled with the sequences of 5'gaaacagctgaatggacccaa3'. Primers were synthesized by MWG in the USA.
2). Primer extension:
   The primer extension was performed using Deep Vent+ and Deep Vent- respectively with the allele specific forward primers and reverse primer. The reaction conditions were: denature at 95 °C for 5 minutes, then cycled 25 times with denature at 95 °C for 10 seconds, annealing at 58.9 °C for 30 seconds, extension 72 °C for 30 second.
3). Results:
   PCR results were under electrophoresis with 2.5% agarose gel containing ethidium bromide under 10V/cm direct current, with 0.5 X TBE butter. PCR products were cut from the gel, and the isotopic activities were counted by liquid scintillation counter. As shown in Figure 5, little radioactivity was detected from isotopic labeled primers when the amplicon was mismatched, demonstrating the removal of the labeled terminal nucleotide by 3' to 5' exonuclease digestion, only 91 cpm in a similar level as control. However, the radioactivity from matched amplicon was 1019 cpm, more than 10 times higher than control.

The results from this experiment demonstrated that products from matched amplicons had detectible isotopic signals. However, when primers and templates were not matched, isotopic labels at the 3' terminal of the primers were eliminated by the 3' to 5' exonuclease digestion, thus the products did not have high value of radioactivity.

In addition to 3[H] labeling, Deep Vent+ polymerase together with fluoresecent labeled primers such as Rox labeling also showed very efficient discrimination of SNP. Based on the 3' terminal labeled primer study, we demonstrated the application of mismatch proofreading mechanism by high fidelity polymerases such as Deep Vent+ can be well used in SNP assay.

### Example 3.

Sequencing analysis using polymerases with 3' to 5' exonuclease activity together with the phosphorothioate-modified matched and mismatched primers.
1). Primer design:
   Using a 217 bp fragment from mouse genome with the following sequences: cccaagatat ctgagaattc tcagcagcct tccatttaga agggtgttgt tgtctctgag gcaaaaccac atttcttacc gcacaactag agactgagac cagtttctct cattgtcatt gctgctcaga gccagcagaa aagcactcat gacacacact tagaataata gtgcatctga gccaggactg cccttggggt ccattcagct gtttc. Design 7 forward primers and a reverse primer (a primer set) targeting this sequence. The forward primers contain one perfectly matched primer and 6 one base mismatched allele specific priemrs (forward primer subset), size of the product from these primers is 217 bp.
   Perfectly matched primers has the sequence of 5'atcccaagatatctgagaattc3';
   The capital letter in the following primers were the mismatched nucleotides from -1 to -6 upstream of the 3' termini: 
5'atcccaagatatct gagaattG3'
5'atcccaagatatctgagaatAc3'
5'atcccaagatatctgagaaAtc3'
5'atcccaagatatctgagaTttc3'
5'atcccaagatatctgagTattc3'
5'atcccaagatatctgaCaattc3'
   All forward primers are phosphorothioate-modified at their 3' termini.
   Reverse primers is not phosphorothioate-modified, its sequence is 5'gaaacagctgaatggacccaa3'. Primers were synthesized by MWG Inc. in USA.
2). Primer extension:
   The primer extension was performed using Deep Vent+ and Deep Vent- respectively with the allele specific forward primers and reverse primer. The reaction conditions were: denature at 95 °C for 5 minutes, then cycled 25 times with denature at 95 °C for 40 seconds, annealing at 58 °C for 30 seconds, extension 72 °C for 30 second.
3). Results:
   PCR results were under electrophoresis with 2.5% agarose gel containing ethidium bromide under 10V/cm direct current, with 0.5 X TBE butter. As shown in Figure 7, matched primers were extended no matter the type of polymerases used. However, phosphorthiolate-modified mismatched primers did not extended by polymerases with 3' to 5' exonucleases, but they were extended by exo-polymerases without 3' to 5' proofreading activity. These results decoded the GAATTC from the template to be assayed.

The results from this experiment demonstrated that products from matched amplicons were not altered by Deep Vent-, thus the 3' terminal nucleotides kept in the products without digestion. Therefore, the 3' terminal phosphorothioate modification did not affect PCR reaction mediated by Deep Vent-. When Deep Vent+ polymerases with 3' to 5' exonuclease activity was used, mismatched nucleotides at 3' or near 3' termini with phosphorothioate modification cannot be rapidly digested by 3' to 5' exonuclease as those modification offered exonuclease-resistance feature to the primer, leading to a premature termination of DNA replication, that was an "off" switch effect. Therefore, mismatched primers with phosphorothioate modification were not extended by high fidelity DNA polymerases with 3' to 5' exonuclease function., and this method is useful in sequencing analysis in this way had detectible isotopic signals.

### Example 4.

SNP analysis using polymerases with 3' to 5' exonuclease activity together with the phosphorothioate-modified matched and mismatched primers (see figure 6).
1). Primer design:
   Using a 217 bp fragment from mouse genome with the following sequences: cccaagatat ctgagaattc tcagcagcct tccatttaga agggtgttgt tgtctctgag gcaaaaccac atttcttacc gcacaactag agactgagac cagtttctct cattgtcatt gctgctcaga gccagcagaa aagcactcat gacacacact tagaataata gtgcatctga gccaggactg cccttggggt ccattcagct gtttc. Design 2 forward primers and one reverse primer. Forward primers were allele specific, including one matched and one mismatched primers. Matched primers has the sequence of 5' atcccaagatatctgagaattc3'; the 3' mismatched primers has the sequence of 5' atcccaagatatctgagaattG3'. The both forward primers were phosphorothioate modified. Reverse primer was not modified, its sequence is 5' gaaacagctgaatggacccaa3'. The size of the product from these primers is 217 bp. Primers were synthesized by MWG Inc. in USA.
2). Primer extension:
   The primer extension was performed using Deep Vent+ and Deep Vent- respectively with the allele specific forward primers and reverse primer. The reaction conditions were: denature at 95 °C for 5 minutes, then cycled 25 times with denature at 95 °C for 40 seconds, annealing at 58 °C for 30 seconds, extension 72 °C for 30 second.
3). Results:
   PCR results were under electrophoresis with 2.5% agarose gel containing ethidium bromide under 10V/cm direct current, with 0.5 X TBE butter. As shown in Figure 6, accurate and reliable primer extension was observed in primer extension using polymerases with 3' to 5' exonuclease activity in annealing temperatures differed from each other more than 10 degrees. Mismatched primers were not extended by high fidelity polymerases. However, with the Deep Vent-polymerases without 3' to 5' exonuclease, discrimination of SNP only observed at annealing temperature higher than 62 °C. This experiment illustrated that the method of this invention can be used to decode sequence information in same or similar conditions required by many primers or multiplex conditions.

### Example 5.

Detection of C→T point mutation in sensual deaf GJB3 gene using pfu polymerase with 3' to 5' exonuclease activity together with matched and mismatched phosphorothioate-modified primers.
1). Primer design:
   Targeting the GJB3 gene's wild type C allele and mutant. The forward primer is 5' caa cat cgt gga ctg cta cat tgc cc3'. The reverse primer is 5' gtg aag att ttc ttc ttg gta ggt cg3'. Deafness mutant T allele was targeted by forward primers of 5' caa cat cgt gga ctg cta cat tgc ct', with reverse primers of 5' gtg aag att ttc ttc ttg gta ggt ca3'. The forward primers are phosphorothioate modified. Primers were synthesized by Sangon Inc. at Shanghai, China.
2). Primer extension.
   The primer pairs were used in PCR with pfu and taq, the latter has no 3' to 5' exonuclease activity. Primer extension was performed under the condition: denature at 95 °C for 5 minutes, then cycled 30 times with denature at 95 °C for 40 seconds, annealing at 56 °C for 40 seconds, extension 72 °C for 30 second.
3). Results.

PCR results were under electrophoresis with 2.5% agarose gel containing ethidium bromide under 10V/cm direct current, with 0.5 X TBE butter. As shown in Figure 8,
When using taq that has no 3' to 5' exonuclease function, both primers targeting wild type allele and mutant allele were extended with the application of wild type homozygous DNA as template. The only difference is that mismatched primer yielded relatively less amount of products. As show in figure 9, the pfu polymerases with 3' to 5' exonuclease activity has high ability to distinguish single base mismatch. Primers targeting the wild type allele is perfectly matched with the template were extended by pfu. However, primers targeting the mutant allele was one base mismatched with the wild type genomic DNA were not extended by pfu, no products observed then.

Primer extension with the employment of phosphorothioate modified primers together with pfu having 3' to 5' exonuclease activity, can rapidly and accurately analyze genomic DNA at single nucleotide level.

## Claims

1. A method utilizing the high fidelity polymerases with 3'to 5'exonuclease activity for sequence determination, including the following steps:
a). allele specific primers with their 3'termini specifically modified,
b). performing a selective primer extension for the discrimination of matched and mismatched amplicons by using a novel ön/off" switch consisting the polymerases with 3'to 5'exonucleases activities to destroy primers from 3'to 5'and primers with exonuclease-resistant characters inherited from specific chemical modification,
c). sequence determination by visualization of the primer extended products;

2. A method according to claim 1, the allele specific primers are different in their 3'termini;

3. A method according to claim 1, the primer set consists of chemically modified and unmodified primers;

4. A method according to claim 3, the chemically modified primers are 3'to 5'exonuclease-sensitive but labeled or nuclease-resistant;

5. A method according to claim 1, the molecular "on/off" switch can "off" (eliminate) the label from nuclease-sensitive primer for the mismatched amplicons and 'on' the labeled for the matched amplicons; or turns off the mismatched amplicon to the nuclease resistant primers in the mismatched amplicon and turns on the primer extension to the nuclease-resistant primers for the matched amplicons;

6. A method according to claim 3, the visualization of primer extension product can use routine methods for DNA, or the visualization can be specific based on whether the labeling is kept of not in the products;

7. A method according to claim 3, the visualization of primer extension products can directly use the characteristic labels inherited from the labeled primers, or use additional labels such as from labeled dNTPs.
